# EUROPEAN PATENT APPLICATION

(11) **EP 4 769 301 A1**
(43) Date of publication of application: **01.07.2026**
(21) Application number: 25223061.0
(22) Date of filing: 12.12.2025
(51) Int. Cl.: G06T 7/00

(54) **METHOD FOR DETECTING ABNORMAL REGION IN IMAGE, METHOD FOR BASE CALLING BY PROCESSING ABNORMAL REGION IN IMAGE, AND RELATED DEVICE**

(30) Priority: 30.12.2024 CN 202411990489
(71) Applicant: GeneMind Biosciences Company Limited, Shenzhen, Guangdong 518023 (CN); Nanjing GeneMind Biosciences Company Limited, Nanjing City, Jiangsu 210000 (CN)
(72) Inventor: Li, Linsen, Shenzhen City, 518023 (CN)
(74) Representative: Schiweck Weinzierl Koch Patentanwälte Partnerschaft mbB

(57) **Abstract**

The present application relates to the field of sequencing image data processing technology, and in particular, to a method for detecting an abnormal region in an image, a method for base calling by processing an abnormal region in an image, and a related device. In the present application, the abnormal region is identified by analyzing the intensity of the pixel where the position of the nucleic acid template is located in the image of interest and the brightness of image blocks in the image of interest, and the intensity of the position of the nucleic acid template in the abnormal region is zeroed, so as to identify the base type of the nucleic acid template incorporated into the abnormal region in the sequencing process as unknown, thereby eliminating the abnormal signal interference and improving the accuracy of base sequencing.

## Description

### TECHNICAL FIELD

The present application relates to the field of sequencing image data processing technology, and in particular, to a method for detecting an abnormal region in an image, a method for base calling by processing an abnormal region in an image, and a related device.

### BACKGROUND

In the process of high-precision gene sequencing, it is necessary to accurately identify and analyze weak signals on the sequencing chip. However, the actual sequencing process often faces various interferences and abnormal conditions, such as dust and dirt on the chip surface, substrate shedding and abnormal adsorption within the chip, and bubbles in the fluid. Such abnormalities may cause problems such as blurry sequencing images, signal masking, and base calling errors.

Therefore, in order to reduce the influence of the abnormal regions in the images on the sequencing throughput and sequencing accuracy, it is necessary to identify and process the abnormal region in the image.

### SUMMARY

In view of the above problems, the present application provides a method for detecting an abnormal region in an image, a method for base calling by processing an abnormal region in an image, and a related device, which overcome or at least partially solve the above problems. The technical solutions are as follows:
The embodiments of the present application provide a method for detecting an abnormal region in an image, including:
acquiring an image of interest, the image of interest being generated by imaging during the sequencing of a nucleic acid template on a surface of a solid substrate;
determining a position of the nucleic acid template in the image of interest;
determining an intensity of a pixel where the nucleic acid template in the image of interest is located on the basis of the position of the nucleic acid template in the image of interest;
determining whether a signal is present at the position of the nucleic acid template in the image of interest on the basis of the intensity; and
using a region formed at a position where the nucleic acid template produces no signal in the image of interest as an abnormal region of the image of interest,
where the signal is generated during sequencing of the nucleic acid template.

The embodiments of the present application provide a method for detecting an abnormal region in an image, including:
acquiring an image of interest, the image of interest being generated by imaging during the sequencing of a nucleic acid template on a surface of a solid substrate, a signal generated during the sequencing of the nucleic acid template being present as a spot in the image of interest, and the brightness of the image of interest being associated with an intensity of the spot;
dividing the image of interest into a number of image blocks;
determining an evaluation value of each image block; and
determining whether the image block is an abnormal region of the image of interest on the basis of the evaluation value of the image block,
where the evaluation value is associated with the brightness of the image block.

The embodiments of the present application provide a method for base calling by processing an abnormal region in an image, including:
determining an abnormal region in the image by detecting with the method for detecting an abnormal region in an image according to any one of the above items;
determining a position of the nucleic acid template in the abnormal region in the image of interest; and
when performing base calling on the basis of the image of interest, zeroing an intensity of a position where the nucleic acid template is located in the abnormal region in the image of interest, and identifying a base type corresponding to the position where the nucleic acid template is located in the abnormal region as an unknown base.

A computer-readable storage medium having a program stored thereon, where the program is executable by a processor to implement the method for detecting an abnormal region in an image described above and/or the method for base calling by processing an abnormal region in an image described above.

A system, including:
the computer-readable storage medium described above; and
at least one processor configured for executing a program stored in the computer-readable storage medium.

An electronic device, including at least one processor, at least one memory connected to the processor, and a bus, where the processor and the memory communicate with each other through the bus; the processor is configured to invoke program instructions in the memory to execute the method for detecting an abnormal region in an image described above and/or the method for base calling by processing an abnormal region in an image described above.

A computer program product, including: a first instruction for detecting an abnormal region in an image and/or a second instruction for base calling by processing the abnormal region in the image, where the first instruction, when executed by a computer, causes the computer to execute the method for detecting an abnormal region in an image described above, and the second instruction, when executed by a computer, causes the computer to execute the method for base calling by processing an abnormal region in an image described above.

By means of the above technical solutions, the present application provides a method for detecting an abnormal region in an image, a method for base calling by processing an abnormal region in an image, and a related device. The method includes: firstly, acquiring an image of interest generated by imaging a nucleic acid template on a surface of a solid substrate, and identifying an abnormal region by analyzing an intensity of a pixel where a position of the nucleic acid template in the image of interest is located and/or and the brightness of image blocks in the image of interest; and secondly, when the abnormal region in the image is processed, zeroing the intensity of the position of the nucleic acid template in the abnormal region, and identifying the base type of the nucleic acid template incorporated into the abnormal region during sequencing as unknown, so as to ensure the accuracy of subsequent base calling. It can be seen that the present application can effectively identify and process abnormal regions, thereby improving the accuracy and reliability of sequencing data.

The above description is only an overview of the technical solutions of the present application. In order to understand the technical means of the present application more clearly, the present application can be implemented according to the contents of the specification, and in order to make the above and other objectives, features, and advantages of the present application more apparent and understandable, specific embodiments of the present application are provided below.

### BRIEF DESCRIPTION OF THE DRAWINGS

Various other advantages and benefits will become apparent to those of ordinary skill in the art upon reading the following detailed description of the preferred embodiments. The drawings are only for the purpose of illustrating the preferred embodiments and should not be construed as limiting the present application. In addition, the same reference numerals are used to represent the same components throughout the drawings. In the drawings:
FIG. 1 illustrates a schematic flowchart of one embodiment of the method for detecting an abnormal region in an image according to the examples of the present application;
FIG. 2 illustrates a schematic flowchart of another embodiment of the method for detecting an abnormal region in an image according to the examples of the present application;
FIG. 3 illustrates a schematic flowchart of another embodiment of the method for detecting an abnormal region in an image according to the examples of the present application;
FIG. 4 illustrates a schematic flowchart of another embodiment of the method for detecting an abnormal region in an image according to the examples of the present application;
FIG. 5 illustrates a schematic view of the layout of structural elements in an image of interest according to the examples of the present application;
FIG. 6 illustrates a schematic flowchart of one embodiment of the method for base calling by processing an abnormal region in an image according to the examples of the present application; and
FIG. 7 illustrates a schematic view of an electronic device according to the examples of the present application.

### DETAILED DESCRIPTION

Illustrative examples of the present application will be described in more detail below with reference to the drawings. Although illustrative examples of the present application are shown in the drawings, it will be appreciated that the present application may be implemented in various forms and is not limited by the examples set forth herein. Rather, such examples are provided that the present application will be thoroughly understood and the scope of the present application can be completely clarified for those skilled in the art.

It should be noted that, as used in this specification and the appended claims, the singular form "a", "an" and "the" include plural referents unless the content clearly dictated otherwise. Thus, for example, reference to "a nucleic acid template" includes two or more nucleic acid templates, and the like.

It should be noted that the term "solid substrate" may be any solid support useful for immobilizing nucleic acid sequences, such as nylon membranes, glass slides, plastics, silicon wafers, magnetic beads, and the like. Unless otherwise specified, the surface of a sequencing chip and the surface of a solid substrate are interchangeable in description.

The term "sequencing" is also referred to as "nucleic acid sequencing" or "gene sequencing" (i.e., the three expressions are interchangeable) and refers to the determination of the type and order of bases in a nucleic acid sequence, including sequencing by synthesis (SBS), sequencing by ligation (SBL) and/or the like, including DNA sequencing and/or RNA sequencing, including long fragment sequencing and/or short fragment sequencing (the long fragment and short fragment are defined relatively; for example, nucleic acid molecules longer than 1 Kb, 2 Kb, 5 Kb, or 10 Kb may be referred to as long fragments, and nucleic acid molecules shorter than 1 Kb or 800 bp may be referred to as short fragments), and including double-end sequencing, single-end sequencing, paired-end sequencing, and/or the like (the double-end sequencing or paired-end sequencing may refer to the reading of any two segments or portions of the same nucleic acid template that are not completely overlapping); the sequencing involves the process of binding nucleotides or nucleotide analogs to the nucleic acid template and acquiring the corresponding signals emitted upon binding the nucleotides or nucleotide analogs to the nucleic acid template.

Sequencing generally involves multiple cycles of processes to achieve the determination of the order of multiple nucleotides/bases on the nucleic acid template, and each cycle of "a process to achieve the determination of the order of multiple nucleotides/bases on the nucleic acid template" may be referred to as "one cycle of sequencing" in the examples of the present application. One "cycle of sequencing", also referred to as "sequencing cycle", may be defined as completion of the base extensions of four types of nucleotides/bases once, and in other words, one "cycle of sequencing" may be defined as the determination of the base type at any given position on the nucleic acid template. For sequencing platforms that achieve sequencing on the basis of polymerization or ligation reactions, one cycle of sequencing includes the process of binding four types of nucleotides (including nucleotide analogs) to the nucleic acid template at a time and acquiring the corresponding signals emitted; for platforms that achieve sequencing on the basis of polymerization reaction, the reaction system includes reaction substrate nucleotides or nucleotide analogs, a polymerase, and a nucleic acid template, a sequence fragment (a sequencing primer) is bound to the nucleic acid template, and on the basis of the principles of base pairing and polymerization reaction, the added reaction substrate nucleotides or nucleotide analogs are connected to the sequencing primer under the catalysis of the polymerase to achieve the binding of the nucleotides or nucleotide analogs to a specific position of the nucleic acid template, that is, the nucleotides or nucleotide analogs are incorporated into the nucleic acid template. Generally, one cycle of sequencing may include one or more base extensions (repeats). For example, four types of nucleotides are sequentially added to the reaction system to each perform base extension and corresponding acquisition of reaction signals, and one cycle of sequencing includes four base extensions; for another example, four types of nucleotides are added into the reaction system in any combinations (such as in pairs or in one-three combinations), the two combinations each perform base extension and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension. The term "nucleic acid template" may refer to a polymeric form of nucleotides of any length, and may include ribonucleotides, deoxyribonucleotides, analogs thereof, or mixtures thereof. The term may refer to a single-stranded or double-stranded polynucleotide. Nucleotides in a nucleic acid template may include naturally occurring nucleotides and functionally alternative analogs thereof. Examples of analogs can hybridize to nucleic acids in a sequence-specific manner, or can be used as templates for the replication of particular nucleotide sequences. Naturally occurring nucleotides generally have a backbone containing a phosphodiester bond. Analog structures may have alternative backbone linkages including any types known in the art. Natural nucleotides generally have deoxyribose (e.g., found in DNA) or ribose (e.g., found in RNA). Analog structures may have alternative sugar moieties including any types known in the art. Nucleotides may contain natural bases. Bases in natural DNA may include one or more of adenine, thymine, cytosine, and/or guanine, and bases in natural RNA may include one or more of adenine, uracil, cytosine, and/or guanine. Any non-natural base or base analog may also be contained in a nucleotide, such as a locked nucleic acid (LNA) and a bridged nucleic acid (BNA).

The term "template image" may be, for example, an image constructed on the basis of one or more cycles of sequencing of the nucleic acid template and configured for characterizing a position of the nucleic acid template on the surface of the solid substrate, which includes position information of nucleic acid templates on the surface of the solid substrate. For methods for constructing the template image, refer to Chinese Patent No. CN112288783B.

In the second-generation or third-generation sequencing technology, it is important to accurately identify and analyze weak signals on the surface of the sequencing chip during sequencing. The weak signal is, for example, a fluorescence signal generated by exciting a fluorophore carried by a base incorporated into a nucleic acid template by excitation light during sequencing. However, in the actual sequencing process, problems such as dust and dirt on the sequencing chip surface, substrate shedding within the sequencing chip, abnormal adsorption, and bubbles in the fluid are often encountered. These problems lead to abnormalities in the images of the sequencing chip during the sequencing process, such as blurred images or masked signals in the images, thereby causing information identification errors. Image abnormalities may lead to base calling errors, including base type calling errors and deviations in sequencing quality scores. These errors and deviations, especially base type identification errors with high sequencing quality scores, may affect subsequent bio-information analysis and ultimately lead to deviations in analysis results. Therefore, when an abnormal region of the image is detected, further processing must be performed to minimize the impact of the abnormal region on the sequencing data and ensure the accuracy and reliability of the sequencing data analysis.

On this basis, the examples of the present application provide a method for detecting an abnormal region in an image and a method for base calling by processing an abnormal region in an image. First, an image of interest obtained by imaging a nucleic acid template on a surface of a solid substrate is acquired, and an abnormal region in the image of interest is identified by analyzing an intensity of a pixel where a position of the nucleic acid template in the image of interest is located and/or and the brightness of image blocks in the image of interest. These abnormal regions are usually present as, e.g., black spots, white spots, or blurred regions. Secondly, when these abnormal regions are processed, by zeroing an intensity of a position where the nucleic acid template is located in the abnormal region and marking the type of bases incorporated into the nucleic acid template in the abnormal region during sequencing as unknown, the accuracy of subsequent base calling can be ensured. It can be seen that the present application can effectively identify and process abnormal regions, thereby significantly improving the accuracy and reliability of sequencing data.

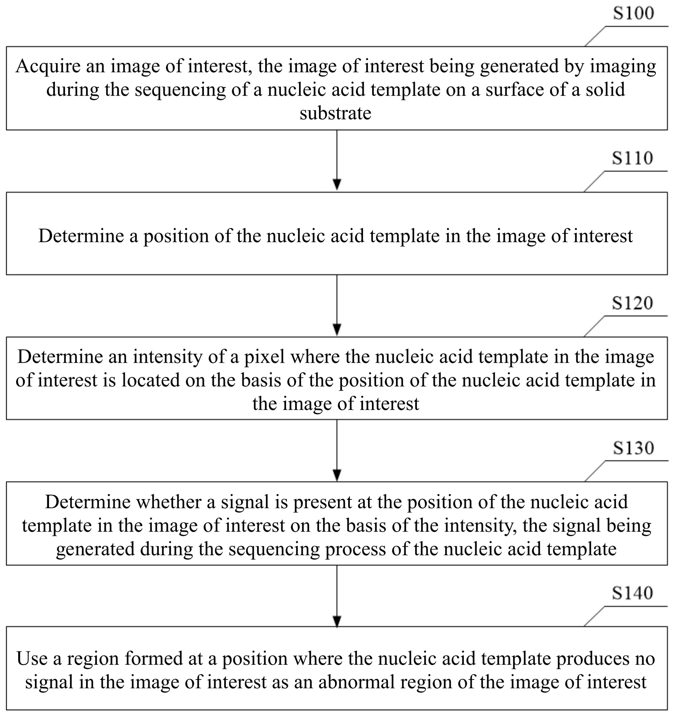

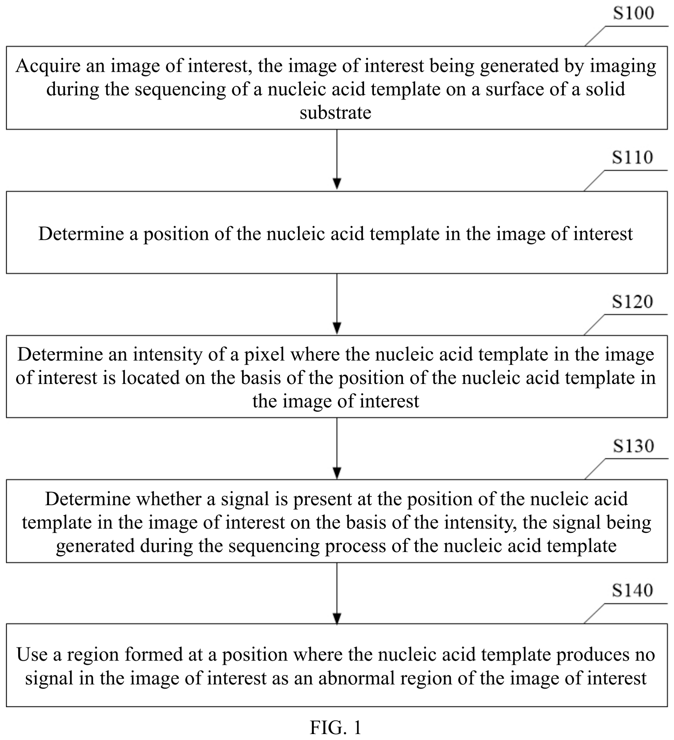

FIG. 1 shows a schematic flowchart of one embodiment of the method for detecting an abnormal region in an image according to the examples of the present application. The method may include:
S100, acquiring an image of interest, the image of interest being generated by imaging during the sequencing of a nucleic acid template on a surface of a solid substrate.

The nucleic acid template is bound to a sequencing primer (e.g., an oligonucleotide) immobilized on the surface of the solid substrate. In a sequencing process, such as a sequencing by synthesis (SBS)-based sequencing process, under the action of a DNA polymerase and in a condition suitable for the polymerase chain reaction, a nucleotide or a nucleotide analog with a fluorophore and a cleavable blocking group is incorporated into the nucleic acid template, so as to perform single base extension on the sequencing primer and excite the fluorophore on the nucleotide or the nucleotide analog to generate a fluorescence signal. By imaging the surface of the solid substrate with an optical imaging system and acquiring the fluorescence signal on the surface of the solid substrate to give an image, the type of the base incorporated into the nucleic acid template can be obtained on the basis of the analysis of the image. After multiple cycles of sequencing and sequential reading of the types of incorporated bases, the nucleotide sequence of the nucleic acid template is obtained. The image of interest is an image formed by imaging the surface of the solid substrate with an optical imaging system and acquiring fluorescence signals on the surface of the solid substrate during sequencing. The fluorescence signals are generally present as spots or peaks in the image. That is, the spots or peaks in the image of interest correspond to the fluorescence signal at the position where the nucleic acid template is located on the surface of the solid substrate.

S110, determining a position of the nucleic acid template in the image of interest.

Illustratively, in the examples of the present application, the position of the nucleic acid template in the image of interest can be determined using known nucleic acid template coordinates or according to special marks designed on the surface of the solid substrate. Illustratively, the position of the nucleic acid template in the image of interest can be represented in the form of two-dimensional coordinates to indicate the exact position of the nucleic acid template in the two-dimensional data matrix presented by the image of interest.

S120, determining an intensity of a pixel where the nucleic acid template in the image of interest is located on the basis of the position of the nucleic acid template in the image of interest.

Specifically, in the examples of the present application, an image processing technique can be used to extract an intensity at the position where the nucleic acid template is located in the image of interest, where the intensity is configured to characterize the brightness information of the pixel corresponding to the position where the nucleic acid template is located in the image of interest.

S130, determining whether a signal is present at the position of the nucleic acid template in the image of interest on the basis of the intensity, the signal being generated during the sequencing process of the nucleic acid template.

Illustratively, in the examples of the present application, whether an intensity difference between the intensity of the pixel where the position of the nucleic acid template in the image of interest is located and the intensity of a neighborhood pixel exceeds a preset intensity threshold may be determined. If yes, it is determined that a signal is present at the position of the nucleic acid template in the image of interest; otherwise, it is determined that no signal is present at the position of the nucleic acid template in the image of interest.

S140, using a region formed at a position where the nucleic acid template produces no signal in the image of interest as an abnormal region of the image of interest.

Specifically, if no signal is detected at a position corresponding to the nucleic acid template in the image of interest, the position is included in the abnormal region of the image of interest.

In the examples of the present application, the surface of the solid substrate is imaged during nucleic acid sequencing to give the image of interest, the positions of the nucleic acid templates in the image of interest are identified, the presence or absence of signals is determined on the basis of the intensities of these positions, and the positioned signal-free regions are marked as abnormal regions, which is beneficial to automatically identifying the absence of signals or error signals and improving the accuracy and reliability of sequencing data.

In some examples, on the basis of one or more examples corresponding to FIG. 1, in another optional example provided by the examples of the present application, determining the position of the nucleic acid template in the image of interest may specifically include:
acquiring a template image, the template image being an image constructed on the basis of one or more cycles of sequencing of the nucleic acid template and configured for characterizing a position of the nucleic acid template on the surface of the solid substrate; and mapping the position of the nucleic acid template to the image of interest to determine the position of the nucleic acid template in the image of interest. Illustratively, in the examples of the present application, the nucleic acid template on the solid substrate may be sequenced by using the SBS sequencing technology. During the sequencing, fluorophores carried by nucleotides or nucleotide analogs incorporated into the nucleic acid template generate fluorescence signals under the excitation by excitation light. By using an optical imaging system to acquire fluorescence signals and form an image, the position of the nucleic acid template on the surface of the solid substrate can be determined on the basis of the fluorescence signals in the image.

After one or more cycles of sequencing, a set of spots or peaks corresponding to the nucleic acid template obtained by combining the spots or peaks in the images acquired from the one or more cycles of sequencing can be used, so as to give a template image for indicating the position of the nucleic acid template on the surface of the solid substrate. Then, the template image is analyzed using image processing techniques to detect and mark the position coordinates of each spot or peak corresponding to the nucleic acid template. Finally, the coordinates of positions marked in the template image are mapped to the image of interest using an image registration technique to ensure that the position corresponding to each nucleic acid template can be accurately found in the image of interest.

In the examples of the present application, the position of the nucleic acid template on the surface of the solid substrate can be accurately marked by performing one or more cycles of sequencing on the nucleic acid template to generate a template image. Then, the position information is mapped to a new image of interest, so as to identify the exact position of the nucleic acid template in the image of interest and quickly identify the abnormal regions of the image of interest.

In some examples, on the basis of one or more examples corresponding to FIG. 1, in another optional example provided by the examples of the present application, determining the position of the nucleic acid template in the image of interest may specifically include:
determining a position of a marking element in the image of interest; and determining a position of the nucleic acid template in the image of interest on the basis of the position of the marking element.

The marking element is a visible reference line or pattern for positioning and calibration in the image.

Specifically, in the examples of the present application, an image processing algorithm may be used to identify the position of the marking element in the image of interest. The identified marking element is used as a reference point to calculate precise coordinates of the position of the marking element in the image of interest. Since the relative position of the nucleic acid template to the marking element is generally known, the position of the nucleic acid template in the image of interest is deduced on the basis of the position of the marking element in the image of interest by using the known positional relationship between the nucleic acid template and the marking element.

In the examples of the present application, the marking element in the image of interest is first located, and then the position of the nucleic acid template is accurately deduced on the basis of these known positions, thereby improving the accuracy of locating the nucleic acid template in the image and helping identify and analyze the abnormal regions. In addition, the precise location can reduce errors in the analysis process and ensure more reliable and effective detection of abnormal regions.

In some examples, the marking element includes at least one of a trackline and a crosshair.

The trackline is a continuous line and is used to mark a specific path or position in the image. The trackline can provide a reference for image analysis and help determine the relative position of another element or region in the image.

The crosshair is a mark with a shape similar to "+" and is generally used to mark a key position of an image. The crosshair provides a well-defined crosspoint that can help pinpoint a particular region in the image.

In the examples of the present application, the position of the nucleic acid template in the image can be accurately deduced by finding the position coordinates of the trackline or the crosshair in the image of interest, which helps ensure the accuracy and consistency of subsequent identification of abnormal regions.

In some examples, the marking element may be in a number of geometric forms, such as points, lines, and surfaces (geometric patterns), to adapt to different application requirements and visual recognition requirements. In addition to the conventional tracklines and crosshairs, the marking element may also be an independent spot mark of other different shapes, colors, or patterns, a mark similar to a ring "O", a mark similar to a triangle "Δ", a mark similar to a polygon, etc.

The independent spot mark is a marking symbol for highlighting specific information or regions.

The ring mark is a mark with a shape similar to "O" and is generally used to highlight a specific region, which can provide clear visual guidance in the image and is easy to identify and locate.

The triangle mark is a mark with a shape similar to "Δ", and is generally used to indicate a direction or a specific point of interest.

The polygon mark may be designed in different shapes as desired, such as a pentagon and a hexagon, and is suitable for the marking of a complex image.

In the examples of the present application, a specific point in an image can be accurately positioned by using marking elements in different shapes. In addition, the marking element can not only help improve the readability of the image, but also provide reference points in the imaging process to assist the device in focusing and assessing the imaging quality, thereby more accurately deducing the position of the nucleic acid template in the image and ensuring the accuracy and consistency of subsequent identification of abnormal regions.

In some examples, on the basis of one or more examples corresponding to FIG. 1, in another optional example provided by the examples of the present application, the image of interest includes one or more sets of corresponding sequencing images generated by performing one or more cycles of sequencing on the nucleic acid template.

Using the region formed at the position where the nucleic acid template produces no signal in the image of interest as the abnormal region of the image of interest may specifically include:
using a region formed at a position where the nucleic acid template produces no signal in the one or more sets of sequencing images as the abnormal region.

In the process of sequencing a nucleic acid template, the fluorescence signals generated in all cycles of sequencing are acquired by an optical imaging system to give sequencing images, and such sequencing images are collected to form one or more sets of sequencing images. Each set of images corresponds to the incorporation of a specified nucleotide or nucleotide analog into a particular position of the nucleic acid template.

Specifically, in the examples of the present application, the position where the nucleic acid template produces no signal in one or more sets of sequencing images can be determined by comparing the signal status of the positions where the nucleic acid template is located in the one or more sets of sequencing images, that is, the positions where those nucleic acid templates exhibit no signals in the sequencing images in different sequencing cycles are determined, and the regions formed by these positions are marked as abnormal regions.

In the examples of the present application, by comparing the signal distribution in multiple cycles of sequencing images, abnormal positions that may be caused by mutations, injuries, or experimental errors can be effectively identified and excluded, thereby improving the accuracy of the identification of abnormal regions.

In some examples, on the basis of one or more examples corresponding to FIG. 1, in another optional example provided by the examples of the present application, determining whether the signal is present at the position of the nucleic acid template in the image of interest on the basis of the intensity may specifically include:
determining a difference between the intensity of the pixel where the nucleic acid template in the image of interest is located and an intensity of a neighborhood pixel thereof; comparing the difference with a first threshold; and determining whether a signal is present at the position of the nucleic acid template in the image of interest on the basis of the comparison result.

Specifically, in the examples of the present application, whether a signal is present at the position can be determined by determining the intensity of the pixel where the nucleic acid template in the image of interest is located, and calculating the difference between the intensity thereof and the intensity of a neighborhood pixel.

Furthermore, in the examples of the present application, the calculated difference may be compared with a preset first threshold. If the difference is greater than or equal to the first threshold, it is determined that a signal is present at the position of the nucleic acid template in the image of interest, and the signal is recorded as 1; if the difference is lower than the first threshold, it is determined that no signal is present at the position of the nucleic acid template in the image of interest, and the signal is recorded as 0.

In some examples, the neighborhood includes an N × M neighborhood, where both N and M are odd numbers, N ≥ 3, and M ≥ 3. For example, the intensity of the pixel where the nucleic acid template in the image of interest is located and the neighborhood pixels thereof include edge points or corner points in a 3 × 3 or 5 × 5 neighborhood.

In the examples of the present application, by calculating the difference between the intensity of the nucleic acid template position in the image of interest and the intensity of a neighborhood thereof and comparing the difference with a set threshold to determine whether a signal is present at the position, the signal region of the nucleic acid template in the image can be effectively identified, thereby improving the accuracy and sensitivity of abnormal region detection and helping more accurately identify abnormal regions that may be contained in the image.

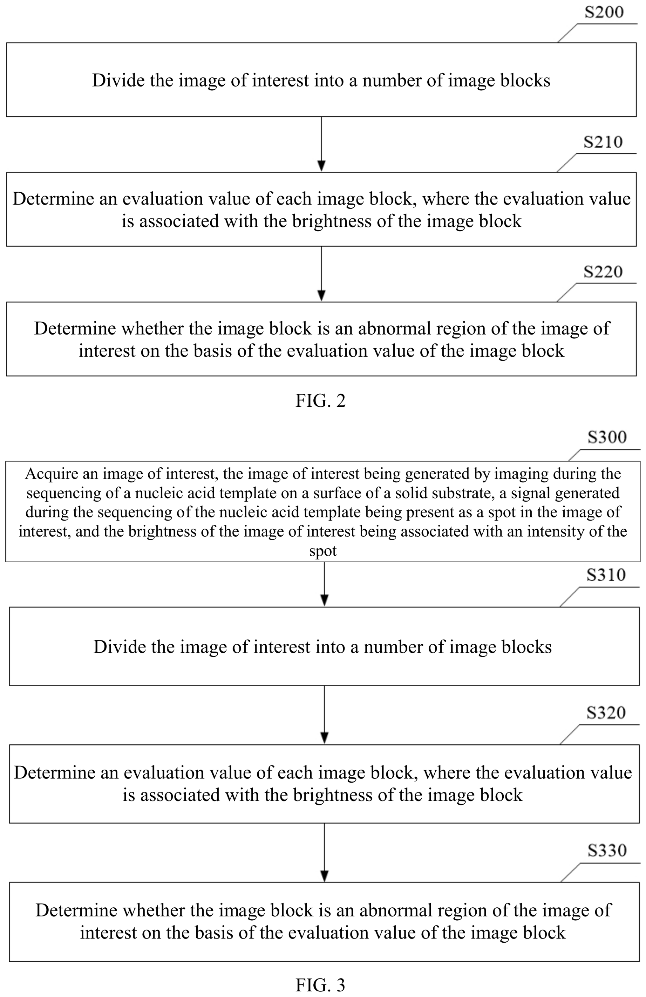

In some examples, on the basis of the method shown in FIG. 1, FIG. 2 shows a schematic flowchart of another embodiment of the method for detecting an abnormal region in an image according to the examples of the present application. The method may further include:
S200, dividing the image of interest into a number of image blocks.

Specifically, in the examples of the present application, the image of interest may be divided into a number of image blocks of the same size on the basis of a preset image block size (for example, 100 × 100 pixels).

S210, determining an evaluation value of each image block, where the evaluation value is associated with the brightness of the image block.

Specifically, in the examples of the present application, for each image block, a mean of the intensities of all pixels in the image block may be calculated, and then the evaluation value of the image block is further calculated by using the mean of the intensities.

S220, determining whether the image block is an abnormal region of the image of interest on the basis of the evaluation value of the image block.

Specifically, in the examples of the present application, an abnormality-determining threshold may be preset, and whether the image block is an abnormal region of the image of interest is determined on the basis of a comparison result between the evaluation value of the image block and the abnormality-determining threshold. Illustratively, the range of the abnormality-determining threshold may be determined according to the global image brightness of the image of interest.

In the examples of the present application, the image of interest is divided into a number of image blocks, the brightness of each image block is evaluated to determine an evaluation value of the image block, and whether the image block is abnormal is determined on the basis of the evaluation value. Therefore, a region with abnormal brightness in the image can be effectively identified, thereby improving the accuracy of detecting abnormal regions, helping quickly locate a defect or an abnormal signal in the image, and enhancing the reliability and efficiency of subsequent image analysis.

In some examples, on the basis of one or more examples corresponding to FIG. 2, in another optional example provided by the examples of the present application, determining the evaluation value of each image block may specifically include:
determining a brightness deviation and a brightness mean of each image block, and determining the evaluation value on the basis of the brightness deviation and the brightness mean.

Specifically, in the examples of the present application, for each image block, a mean of the intensities of all pixels in the image block may be calculated to give a brightness mean, and then the mean of the sum of squares of differences between all pixels in the image block and the brightness mean is calculated to give a brightness deviation (dev) of the image block.

The evaluation value is usually represented by a ratio of the brightness deviation to the brightness mean, that is, evaluation value = brightness deviation/brightness mean. The evaluation value may help normalize the impact of brightness non-uniformity, such that the evaluation value is more stable in image blocks with different brightness.

In the examples of the present application, the brightness deviation and the brightness mean of each image block are determined, and the evaluation value is calculated, such that normal regions and abnormal regions in the image can be effectively distinguished. The brightness mean provides the overall brightness level of the image block, while the brightness deviation measures the degree of brightness fluctuation. A lower evaluation value generally means a smaller brightness fluctuation of the image block, and the image block may be an abnormal region. In the examples of the present application, the calculation of the evaluation value of each image block helps identify a region with uniform brightness and no detailed changes in the image of interest, such that abnormal regions can be more accurately located and marked through subsequent image analysis, thereby improving the detection reliability and accuracy.

In some examples, on the basis of one or more examples corresponding to FIG. 2, in another optional example provided by the examples of the present application, determining whether the image block is an abnormal region of the image of interest on the basis of the evaluation value of the image block may specifically include:
comparing the evaluation value of the image block with a second threshold, where the second threshold is determined on the basis of the global image brightness of the image of interest; and
determining whether the image block is an abnormal region of the image of interest on the basis of a comparison result.

Illustratively, in the examples of the present application, the brightness mean of all pixels in the image of interest may be pre-calculated, so as to give the global image brightness of the image of interest. The second threshold may be specifically adjusted on the basis of the sensitivity requirement of a specific application. For example, the second threshold may be determined by adding a fixed value to or subtracting a fixed value from the global brightness mean, or may be a proportion of the global brightness mean.

Illustratively, in the examples of the present application, the brightness standard deviation of all pixels in the image of interest may be pre-calculated, so as to give the global image brightness of the image of interest. The second threshold may be specifically adjusted on the basis of the sensitivity requirement of a specific application. For example, the second threshold may be determined by adding a fixed value to or subtracting a fixed value from the global brightness standard deviation, or may be a proportion of the global brightness standard deviation.

In the examples of the present application, the evaluation value of each image block may be separately compared with a specified second threshold. If the evaluation value of the image block is lower than the second threshold, it is determined that the image block is an abnormal region of the image of interest. If the evaluation value of the image block is greater than or equal to the second threshold, it is determined that the image block is a non-abnormal region of the image of interest.

In the examples of the present application, the second threshold is dynamically adjusted by using the global image brightness to adapt to different illumination conditions and ensure that identification of abnormal regions is not invalidated due to overall brightness changes or misled by a region with excessive or insufficient brightness. As such, an image block with an evaluation value lower than the second threshold is designated as abnormal, thereby improving the accuracy and flexibility of abnormality detection.

In some examples, on the basis of one or more examples corresponding to FIG. 2, in another optional example provided by the examples of the present application, the image of interest includes one or more sets of corresponding sequencing images generated by performing one or more cycles of sequencing on the nucleic acid template.

If the evaluation value of the image block is lower than the second threshold, the method further includes:
determining whether the evaluation value of the image block in the one or more sets of sequencing images is lower than the second threshold; and
if yes, determining that the image block is an abnormal region of the image of interest.

The sequencing of the nucleic acid template may generate one or more sets of sequencing images. In these sequencing images, the evaluation value of each image block is used to evaluate the brightness change and consistency of the image block. First, the evaluation value of each image block is compared with a second threshold that is set on the basis of the global brightness. If the evaluation value of an image block is lower than the second threshold, it indicates that there may be an abnormality in the sequencing image.

In the examples of the present application, in order to further confirm this abnormality, the performance of the image block with this abnormality in any one of the other sequencing images is verified. If the image block exhibits an evaluation value lower than the second threshold in one or more sets of sequencing images, it indicates that the image block consistently presents abnormal features in different sequencing processes. As such, multiple cycles of sequencing provide additional evidence, making the abnormality detection more reliable and accurate.

In the examples of the present application, by verifying whether the evaluation value of the image block in one or more sets of sequencing images is lower than the second threshold, not only can accidental abnormalities that may occur in a single cycle of sequencing be identified, but also the persistence and consistency of the abnormalities is verified through multiple cycles of sequencing, thereby ensuring the accuracy and stability of the detection of abnormal regions in the images.

In some examples, on the basis of one or more examples corresponding to FIG. 2, in another optional example provided by the examples of the present application, an image expansion operation may be performed on the abnormal region to expand the boundary of the abnormal region.

Specifically, in the examples of the present application, after the abnormal region of the image of interest is detected, an image expansion operation is performed on the abnormal region to expand the boundary pixels of the abnormal region and cover adjacent pixels of the abnormal region, such that the boundary of the abnormal region extends outward.

In the examples of the present application, the boundary of the abnormal region is expanded through the image expansion operation, such that the abnormal region can be more clearly displayed, thereby avoiding missing potential abnormalities near the boundary. In addition, the expansion can connect adjacent scattered abnormal points to form a more complete abnormal region, which helps ensure the accuracy of subsequent base calling.

FIG. 3 shows a schematic flowchart of another embodiment of the method for detecting an abnormal region in an image according to the examples of the present application. The method may include:
S300, acquiring an image of interest, the image of interest being generated by imaging during the sequencing of a nucleic acid template on a surface of a solid substrate, a signal generated during the sequencing of the nucleic acid template being present as a spot in the image of interest, and the brightness of the image of interest being associated with an intensity of the spot.

The nucleic acid template is bound to a sequencing primer (e.g., an oligonucleotide) immobilized on the surface of the solid substrate. In a sequencing process, such as a sequencing by synthesis (SBS)-based sequencing process, under the action of a DNA polymerase and in a condition suitable for the polymerase chain reaction, a nucleotide or a nucleotide analog with a fluorophore and a cleavable blocking group is incorporated into the nucleic acid template, so as to perform single base extension on the sequencing primer and excite the fluorophore on the nucleotide or the nucleotide analog to generate a fluorescence signal. By imaging the surface of the solid substrate with an optical imaging system and acquiring the fluorescence signal on the surface of the solid substrate to give an image, the type of the base incorporated into the nucleic acid template can be obtained on the basis of the analysis of the image. After multiple cycles of sequencing and sequential reading of the types of incorporated bases, the nucleotide sequence of the nucleic acid template is obtained. The image of interest is an image formed by imaging the surface of the solid substrate with an optical imaging system and acquiring fluorescence signals on the surface of the solid substrate during sequencing. The fluorescence signals are generally present as spots or peaks in the image. That is, the spots or peaks in the image of interest correspond to the fluorescence signal at the position where the nucleic acid template is located on the surface of the solid substrate.

S310, dividing the image of interest into a number of image blocks.

Specifically, in the examples of the present application, the image of interest may be divided into a number of image blocks of the same size on the basis of a preset image block size (for example, 100 × 100 pixels).

S320, determining an evaluation value of each image block, where the evaluation value is associated with the brightness of the image block.

Specifically, in the examples of the present application, for each image block, a mean of the intensities of all pixels in the image block may be calculated, and then the evaluation value of the image block is further calculated by using the mean of the intensities.

S330, determining whether the image block is an abnormal region of the image of interest on the basis of the evaluation value of the image block.

Specifically, in the examples of the present application, an abnormality-determining threshold may be preset, and whether the image block is an abnormal region of the image of interest is determined on the basis of a comparison result between the evaluation value of the image block and the abnormality-determining threshold. In some examples, the range of abnormality-determining threshold may be determined according to the global image brightness of the image of interest.

In the examples of the present application, the image of interest is divided into a number of image blocks, the brightness of each image block is evaluated to determine an evaluation value of the image block, and whether the image block is abnormal is determined on the basis of the evaluation value. Therefore, a region with abnormal brightness in the image can be effectively identified, thereby improving the accuracy of detecting abnormal regions, helping quickly locate a defect or an abnormal signal in the image, and enhancing the reliability and efficiency of subsequent image analysis.

In some examples, on the basis of one or more examples corresponding to FIG. 3, in another optional example provided by the examples of the present application, determining the evaluation value of each image block may specifically include:
determining a brightness deviation and a brightness mean of each image block, and determining the evaluation value on the basis of the brightness deviation and the brightness mean.

Specifically, in the examples of the present application, for each image block, a mean of the intensities of all pixels in the image block may be calculated to give a brightness mean, and then the mean of the sum of squares of differences between all pixels in the image block and the brightness mean is calculated to give a brightness deviation (dev) of the image block.

The evaluation value is usually represented by a ratio of the brightness deviation to the brightness mean, that is, evaluation value = brightness deviation/brightness mean. The evaluation value may help normalize the impact of brightness non-uniformity, such that the evaluation value is more stable in image blocks with different brightness.

In the examples of the present application, the brightness deviation and the brightness mean of each image block are determined, and the evaluation value is calculated, such that normal regions and abnormal regions in the image can be effectively distinguished. The brightness mean provides the overall brightness level of the image block, while the brightness deviation measures the degree of brightness fluctuation. A lower evaluation value generally means a smaller brightness fluctuation of the image block, and the image block may be an abnormal region. In the examples of the present application, the calculation of the evaluation value of each image block helps identify a region with uniform brightness and no detailed changes in the image of interest, such that abnormal regions can be more accurately located and marked through subsequent image analysis, thereby improving the detection reliability and accuracy.

In some examples, on the basis of one or more examples corresponding to FIG. 3, in another optional example provided by the examples of the present application, determining whether the image block is an abnormal region of the image of interest on the basis of the evaluation value of the image block may specifically include:
comparing the evaluation value of the image block with a second threshold, where the second threshold is determined on the basis of the global image brightness of the image of interest; and
determining whether the image block is an abnormal region of the image of interest on the basis of a comparison result.

Illustratively, in the examples of the present application, the brightness mean of all pixels in the image of interest may be pre-calculated, so as to give the global image brightness of the image of interest. The second threshold may be specifically adjusted on the basis of the sensitivity requirement of a specific application. For example, the second threshold may be determined by adding a fixed value to or subtracting a fixed value from the global brightness mean, or may be a proportion of the global brightness mean.

Illustratively, in the examples of the present application, the brightness standard deviation of all pixels in the image of interest may be pre-calculated, so as to give the global image brightness of the image of interest. The second threshold may be specifically adjusted on the basis of the sensitivity requirement of a specific application. For example, the second threshold may be determined by adding a fixed value to or subtracting a fixed value from the global brightness standard deviation, or may be a proportion of the global brightness standard deviation.

In the examples of the present application, the evaluation value of each image block may be separately compared with a specified second threshold. If the evaluation value of the image block is lower than the second threshold, it is determined that the image block is an abnormal region of the image of interest. If the evaluation value of the image block is greater than or equal to the second threshold, it is determined that the image block is a non-abnormal region of the image of interest.

In the examples of the present application, the second threshold is dynamically adjusted by using the global image brightness to adapt to different illumination conditions and ensure that identification of abnormal regions is not invalidated due to overall brightness changes or misled by a region with excessive or insufficient brightness. As such, an image block with an evaluation value lower than the second threshold is designated as abnormal, thereby improving the accuracy and flexibility of abnormality detection.

In some examples, on the basis of one or more examples corresponding to FIG. 3, in another optional example provided by the examples of the present application, the image of interest includes one or more sets of corresponding sequencing images generated by performing one or more cycles of sequencing on the nucleic acid template.

If the evaluation value of the image block is lower than the second threshold, the method further includes:
determining whether the evaluation value of the image block in the one or more sets of sequencing images is lower than the second threshold; and
if yes, determining that the image block is an abnormal region of the image of interest.

The sequencing of the nucleic acid template may generate one or more sets of sequencing images. In these sequencing images, the evaluation value of each image block is used to evaluate the brightness change and consistency of the image block. First, the evaluation value of each image block is compared with a second threshold that is set on the basis of the global brightness. If the evaluation value of an image block is lower than the second threshold, it indicates that there may be an abnormality in the sequencing image.

In the examples of the present application, in order to further confirm this abnormality, the performance of the image block with this abnormality in any one of the other sequencing images is verified. If the image block exhibits an evaluation value lower than the second threshold in one or more sets of sequencing images, it indicates that the image block consistently presents abnormal features in different sequencing processes. As such, multiple cycles of sequencing provide additional evidence, making the abnormality detection more reliable and accurate.

In the examples of the present application, by verifying whether the evaluation value of the image block in one or more sets of sequencing images is lower than the second threshold, not only can accidental abnormalities that may occur in a single cycle of sequencing be identified, but also the persistence and consistency of the abnormalities is verified through multiple cycles of sequencing, thereby ensuring the accuracy and stability of the detection of abnormal regions in the images.

In some examples, on the basis of one or more examples corresponding to FIG. 3, in another optional example provided by the examples of the present application, an image expansion operation may be performed on the abnormal region to expand the boundary of the abnormal region.

Specifically, in the examples of the present application, after the abnormal region of the image of interest is detected, an image expansion operation is performed on the abnormal region to expand the boundary pixels of the abnormal region and cover adjacent pixels of the abnormal region, such that the boundary of the abnormal region extends outward.

In the examples of the present application, the boundary of the abnormal region is expanded through the image expansion operation, such that the abnormal region can be more clearly displayed, thereby avoiding missing potential abnormalities near the boundary. In addition, the expansion can connect adjacent scattered abnormal points to form a more complete abnormal region, which helps ensure the accuracy of subsequent base calling.

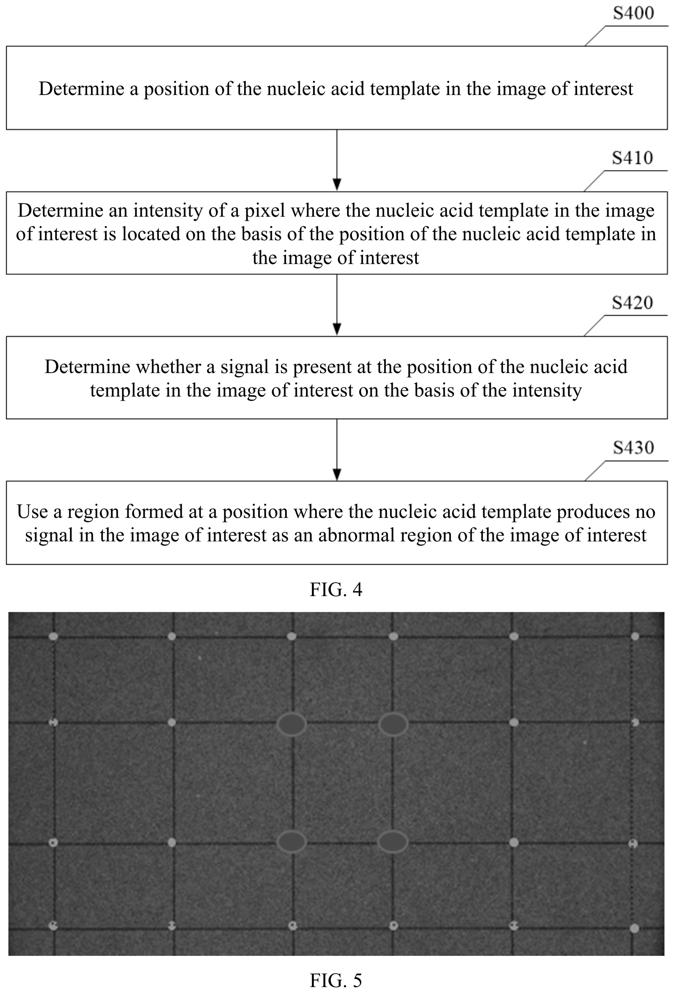

In some examples, on the basis of the method shown in FIG. 3, FIG. 4 shows a schematic flowchart of another embodiment of the method for detecting an abnormal region in an image according to the examples of the present application. The method may further include:
S400, determining a position of the nucleic acid template in the image of interest.

Illustratively, in the examples of the present application, the position of the nucleic acid template in the image of interest can be determined using known nucleic acid template coordinates or according to special marks designed on the surface of the solid substrate. Illustratively, the position of the nucleic acid template in the image of interest can be represented in the form of two-dimensional coordinates to indicate the exact position of the nucleic acid template in the two-dimensional data matrix presented by the image of interest.

S410, determining an intensity of a pixel where the nucleic acid template in the image of interest is located on the basis of the position of the nucleic acid template in the image of interest.

Specifically, in the examples of the present application, an image processing technique can be used to extract an intensity at the position where the nucleic acid template is located in the image of interest, where the intensity is configured to characterize the brightness information of the pixel corresponding to the position where the nucleic acid template is located in the image of interest.

S420, determining whether a signal is present at the position of the nucleic acid template in the image of interest on the basis of the intensity.

Illustratively, in the examples of the present application, whether an intensity difference between the intensity of the pixel where the position of the nucleic acid template in the image of interest is located and the intensity of a neighborhood pixel exceeds a preset intensity threshold may be determined. If yes, it is determined that a signal is present at the position of the nucleic acid template in the image of interest; otherwise, it is determined that no signal is present at the position of the nucleic acid template in the image of interest.

S430, using a region formed at a position where the nucleic acid template produces no signal in the image of interest as an abnormal region of the image of interest.

Specifically, if no signal is detected at a position corresponding to the nucleic acid template in the image of interest, the position is included in the abnormal region of the image of interest.

In the examples of the present application, the surface of the solid substrate is imaged during nucleic acid sequencing to give the image of interest, the positions of the nucleic acid templates in the image of interest are identified, the presence or absence of signals is determined on the basis of the intensities of these positions, and the positioned signal-free regions are marked as abnormal regions, which is beneficial to automatically identifying the absence of signals or error signals and improving the accuracy and reliability of sequencing data.

In some examples, on the basis of one or more examples corresponding to FIG. 4, in another optional example provided by the examples of the present application, determining the position of the nucleic acid template in the image of interest may specifically include:
acquiring a template image, the template image being an image constructed on the basis of one or more cycles of sequencing of the nucleic acid template and configured for characterizing a position of the nucleic acid template on the surface of the solid substrate; and mapping the position of the nucleic acid template to the image of interest to determine the position of the nucleic acid template in the image of interest. Illustratively, in the examples of the present application, the nucleic acid template on the solid substrate may be sequenced by using the SBS sequencing technology. During the sequencing, fluorophores carried by nucleotides or nucleotide analogs incorporated into the nucleic acid template generate fluorescence signals under the excitation by excitation light. By using an optical imaging system to acquire fluorescence signals and form an image, the position of the nucleic acid template on the surface of the solid substrate can be determined on the basis of the fluorescence signals in the image.

After one or more cycles of sequencing, a set of spots or peaks corresponding to the nucleic acid template obtained by combining the spots or peaks in the images acquired from the one or more cycles of sequencing can be used, so as to give a template image for indicating the position of the nucleic acid template on the surface of the solid substrate. Then, the template image is analyzed using image processing techniques to detect and mark the position coordinates of each spot or peak corresponding to the nucleic acid template. Finally, the coordinates of positions marked in the template image are mapped to the image of interest using an image registration technique to ensure that the position corresponding to each nucleic acid template can be accurately found in the image of interest.

In the examples of the present application, the position of the nucleic acid template on the surface of the solid substrate can be accurately marked by performing one or more cycles of sequencing on the nucleic acid template to generate a template image. Then, the position information is mapped to a new image of interest, so as to identify the exact position of the nucleic acid template in the image of interest and quickly identify the abnormal regions of the image of interest.

In some examples, on the basis of one or more examples corresponding to FIG. 4, in another optional example provided by the examples of the present application, determining the position of the nucleic acid template in the image of interest may specifically include:
determining a position of a marking element in the image of interest; and determining a position of the nucleic acid template in the image of interest on the basis of the position of the marking element.

The marking element is a visible reference line or pattern for positioning and calibration in the image. Specifically, in the examples of the present application, an image processing algorithm may be used to identify the position of the marking element in the image of interest. The identified marking element is used as a reference point to calculate precise coordinates of the position of the marking element in the image of interest. Since the relative position of the nucleic acid template to the marking element is generally known, the position of the nucleic acid template in the image of interest is deduced on the basis of the position of the marking element in the image of interest by using the known positional relationship between the nucleic acid template and the marking element.

In the examples of the present application, the marking element in the image of interest is first located, and then the position of the nucleic acid template is accurately deduced on the basis of these known positions, thereby improving the accuracy of locating the nucleic acid template in the image and helping identify and analyze the abnormal regions. In addition, the precise location can reduce errors in the analysis process and ensure more reliable and effective detection of abnormal regions.

In some examples, the marking element includes at least one of a trackline and a crosshair.

The trackline is a continuous line and is used to mark a specific path or position in the image. The trackline can provide a reference for image analysis and help determine the relative position of another element or region in the image.

The crosshair is a mark with a shape similar to "+" and is generally used to mark a key position of an image. The crosshair provides a well-defined crosspoint that can help pinpoint a particular region in the image.

In the examples of the present application, the position of the nucleic acid template in the image can be accurately deduced by finding the position coordinates of the trackline or the crosshair in the image of interest, which helps ensure the accuracy and consistency of subsequent identification of abnormal regions.

In some examples, the marking element may be in a number of geometric forms, such as points, lines, and surfaces (geometric patterns), to adapt to different application requirements and visual recognition requirements. In addition to the conventional tracklines and crosshairs, the marking element may also be an independent spot mark of other different shapes, colors, or patterns, a mark similar to a ring "O", a mark similar to a triangle "Δ", a mark similar to a polygon, etc.

The independent spot mark is a marking symbol for highlighting specific information or regions.

The ring mark is a mark with a shape similar to "O" and is generally used to highlight a specific region, which can provide clear visual guidance in the image and is easy to identify and locate.

The triangle mark is a mark with a shape similar to "Δ", and is generally used to indicate a direction or a specific point of interest.

The polygon mark may be designed in different shapes as desired, such as a pentagon and a hexagon, and is suitable for the marking of a complex image.

In the examples of the present application, a specific point in an image can be accurately positioned by using marking elements in different shapes. In addition, the marking element can not only help improve the readability of the image, but also provide reference points in the imaging process to assist the device in focusing and assessing the imaging quality, thereby more accurately deducing the position of the nucleic acid template in the image and ensuring the accuracy and consistency of subsequent identification of abnormal regions.

In some examples, on the basis of one or more examples corresponding to FIG. 4, in another optional example provided by the examples of the present application, the image of interest includes one or more sets of corresponding sequencing images generated by a process of performing one or more cycles of sequencing on the nucleic acid template.

Using the region formed at the position where the nucleic acid template produces no signal in the image of interest as the abnormal region of the image of interest may specifically include:
using a region formed at a position where the nucleic acid template produces no signal in the one or more sets of sequencing images as the abnormal region.

In the process of sequencing a nucleic acid template, the fluorescence signals generated in all cycles of sequencing are acquired by an optical imaging system to give sequencing images, and such sequencing images are collected to form one or more sets of sequencing images. Each set of images corresponds to the incorporation of a nucleotide or nucleotide analog into a particular position of the nucleic acid template.

Specifically, in the examples of the present application, the position where the nucleic acid template produces no signal in one or more sets of sequencing images can be determined by comparing the signal status of the positions where the nucleic acid template is located in the one or more sets of sequencing images, that is, the positions where those nucleic acid templates exhibit no signals in the sequencing images in different sequencing cycles are determined, and the regions formed by these positions are marked as abnormal regions.

In the examples of the present application, by comparing the signal distribution in multiple cycles of sequencing images, abnormal positions that may be caused by mutations, injuries, or experimental errors can be effectively identified and excluded, thereby improving the accuracy of the identification of abnormal regions.

In some examples, on the basis of one or more examples corresponding to FIG. 4, in another optional example provided by the examples of the present application, determining whether the signal is present at the position of the nucleic acid template in the image of interest on the basis of the intensity may specifically include:
determining a difference between the intensity of the pixel where the nucleic acid template in the image of interest is located and an intensity of a neighborhood pixel thereof; comparing the difference with a first threshold; and determining whether a signal is present at the position of the nucleic acid template in the image of interest on the basis of the comparison result.

Specifically, in the examples of the present application, whether a signal is present at the position can be determined by determining the intensity of the pixel where the nucleic acid template in the image of interest is located, and calculating the difference between the intensity thereof and the intensity of a neighborhood pixel.

Furthermore, in the examples of the present application, the calculated difference may be compared with a preset first threshold. If the difference is greater than or equal to the first threshold, it is determined that a signal is present at the position of the nucleic acid template in the image of interest, and the signal is recorded as 1; if the difference is lower than the first threshold, it is determined that no signal is present at the position of the nucleic acid template in the image of interest, and the signal is recorded as 0.

In some examples, the neighborhood includes an N × M neighborhood, where both N and M are odd numbers, N ≥ 3, and M ≥ 3. For example, the intensity of the pixel where the nucleic acid template in the image of interest is located and the neighborhood pixels thereof include edge points or corner points in a 3 × 3 or 5 × 5 neighborhood.

In the examples of the present application, by calculating the difference between the intensity of the nucleic acid template position in the image of interest and the intensity of a neighborhood thereof and comparing the difference with a set threshold to determine whether a signal is present at the position, the signal region of the nucleic acid template in the image can be effectively identified, thereby improving the accuracy and sensitivity of abnormal region detection and helping more accurately identify abnormal regions that may be contained in the image.

The abnormal region (such as high-brightness noise, a low-brightness black hole, or bubble blurring) in an image may interfere with the base calling process. Such abnormalities may lead to failure in the extraction of information, with the following specific impacts:
Interference with coordinate information: FIG. 5 shows a schematic view of the layout of structural elements in an image of interest according to the examples of the present application. During the image processing, coordinate information such as crosspoints (red points in FIG. 5) of tracklines (black lines in FIG. 5) needs to be extracted to calculate the nucleic acid template coordinates within a block (a rectangular region composed of four red points in FIG. 5). If a crosspoint falls within an abnormal region of an image, the coordinates of the crosspoint may deviate. This causes an error in the calculation of coordinates of normal regions and further affects the accuracy of extracted grayscale information. The grayscale information includes an intensity (Ints).

Interference with grayscale information: Once the coordinate information of the nucleic acid template is obtained, the grayscale information corresponding to all signals of the nucleic acid template on the surface of the solid substrate needs to be extracted. If the signal is located in an abnormal region, the Ints of the extracted signal will be inaccurate.

Since the Ints of the extracted signal needs to be used for correction in a subsequent step, an incorrect Ints may affect the correction of normal data. The correction includes:
Crosstalk correction: the correction of signal interference between different channels. If the Ints of a channel is invalid due to an abnormal region, the crosstalk correction of other channels may be affected.
Phasing correction: the correction of signal time delay between different cycles. If the Ints in a specific cycle is abnormal, the phasing correction may be interfered with.

Normalization: All Ints data in the image are normalized. An abnormal region causes an Ints error in some of the regions, which may affect the normalization and thus the calibration of normal regions.

In summary, abnormal regions in the image may not only cause incorrect nucleic acid template coordinates and Ints, but also amplify their impact during the correction, ultimately resulting in data errors in more normal regions and affecting the overall base calling accuracy. Therefore, after an abnormal region is detected in the image, it is necessary to take measures to limit its influence, prevent the expansion of the abnormality, and eliminate its interference with the base calling.

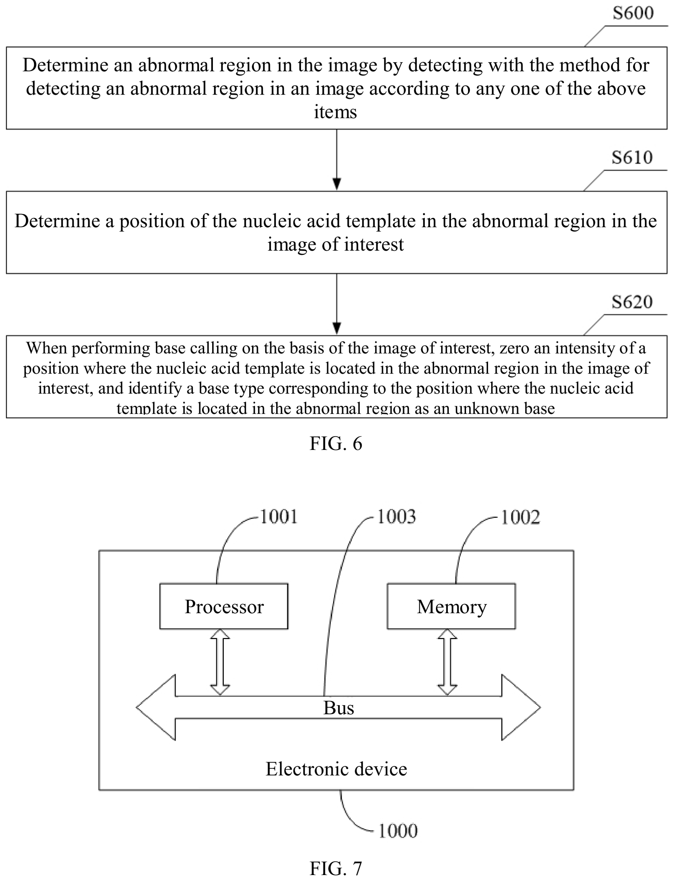

FIG. 6 shows a schematic flowchart of one embodiment of the method for base calling by processing an abnormal region in an image according to the examples of the present application. The method may include:
S600, determining an abnormal region in the image by detecting with the method for detecting an abnormal region in an image according to any one of the above items.
S610, determining a position of the nucleic acid template in the abnormal region in the image of interest. Illustratively, in the examples of the present application, known nucleic acid template coordinates may be directly marked in the image of interest to determine whether the coordinates of the nucleic acid template are located in the abnormal region of the image of interest and further determine the specific positions of the nucleic acid template coordinates located in the abnormal region.

Illustratively, in the examples of the present disclosure, the position of the nucleic acid template in the image of interest may be determined by identifying the special marks designed on the surface of the solid substrate and checking whether the position overlaps with the abnormal region, so as to identify that the overlapping portion is the position of the nucleic acid template in the abnormal region in the image of interest.

S620, when performing base calling on the basis of the image of interest, zeroing an intensity of a position where the nucleic acid template is located in the abnormal region in the image of interest, and identifying a base type corresponding to the position where the nucleic acid template is located in the abnormal region as an unknown base.

Specifically, in the examples of the present application, when base calling is performed on the basis of the image of interest, if the position of the nucleic acid template is in the abnormal region of the image, the intensity of the corresponding position will be directly zeroed. In addition, an additional mark may be provided at these positions to ensure that the impact of these zero values on other normal data is minimized during the correction process. When the bases are finally determined, the bases at these positions are identified as "unknown bases" (denoted by "N") through these marks, indicating that the positions have failed the base calling.

Further, in the examples of the present application, for regions where an abnormality is detected, the intensity of the related location of the region is zeroed, which helps avoid the interference with the calculation of other normal data and the results in a data correction process. The position of each nucleic acid template is provided with a corresponding mark. Even though these positions may be assigned regular intensities and a base may be identified during the correction process, the effect of the mark is to ensure that the position is mandatorily identified as "N" in this case. Since "N" represents an unknown base and will be omitted in subsequent data analysis, the overall error rate will not be increased.

In the examples of the present application, in the image processing process, an abnormal region in the image is detected and determined, then the positions of the nucleic acid templates in the abnormal region are identified, the intensities of these positions are zeroed, and the corresponding base types are marked as unknown base (N), thereby helping reduce the interference with normal data by the abnormal region during the base calling and ensuring the accuracy and reliability of the base calling results. Also, by minimizing the influence of the abnormal region, valid data can be more effectively focused in the base calling process, thereby improving the quality of the overall analysis.

In some examples of the present application, whether data volume or accuracy is prioritized may be selected on the basis of a data processing priority policy. For example, if an abnormal region is found in an image collected by using a channel of the optical imaging system, while the images collected by using the other channels of the optical imaging system are normal, under the accuracy priority policy, the intensities of the signals included in the part corresponding to the abnormal region in all images may be zeroed, that is, the information included in the part corresponding to the abnormal region in normal images is omitted.

For ease of understanding, the data volume priority strategy and the accuracy priority strategy are described herein by means of the following example: It is assumed that during a cycle of multi-channel sequencing, one channel (e.g., a red channel) indicates an abnormal region, while the other channels (e.g., green and blue channels) indicate a normal region. If the data volume priority policy is selected, it means that as much available data as possible is retained, that is, only the intensity of the abnormal region in the red channel is zeroed, and data of the normal channels are retained. This allows normal base calling to continue in the remaining channels, maximizing the use of available data. Finally, the base in the abnormal region in the red channel is marked as "N", while the base calling results of remaining normal channels are still valid. If the accuracy priority policy is selected, it means that data errors are reduced as much as possible, even if this may lead to a reduction in the data volume, that is, all the intensities corresponding to the abnormal region in all the channels are zeroed. This can ensure that the abnormality on any channel does not interfere with the base calling on other channels. Finally, bases corresponding to the positions of the abnormal region in all channels are marked as "N" to avoid misidentification due to asynchrony or crosstalk.

In the examples of the present application, by selecting the data volume priority strategy and the accuracy priority strategy, how to process the abnormal region can be flexibly determined according to the target of base calling and the data environment, so as to acquire optimal base calling results. The choice of strategy often depends on the requirement on data integrity and accuracy of the base calling. In some examples, on the basis of one or more examples corresponding to FIG. 6, in another optional example provided by the examples of the present application, the image of interest includes tracklines, and the tracklines include a number of first tracklines in a first direction and a number of second tracklines in a second direction, where the first direction and the second direction are not parallel, and intersections of the first tracklines and the second tracklines are defined as crosspoints.

Determining a position of the nucleic acid template in the abnormal region in the image of interest may specifically include:
determining coordinates of the crosspoints located in the abnormal region; and calculating coordinates of a position of the nucleic acid template in the abnormal region on the basis of the coordinates of the crosspoints in the abnormal region, to determine the position of the nucleic acid template in the abnormal region in the image of interest.

In the image of interest, there are tracklines of a certain structure: a number of tracklines in a first direction and a number of tracklines in a second direction. The first and second directions are not parallel, so they may intersect. Each intersection is referred to as a "crosspoint". The accurate coordinates of the positions where the nucleic acid templates are located in the abnormal region are further calculated using the identified coordinates of the crosspoints. These coordinates of the positions help determine the specific positions of the nucleic acid templates in the abnormal region of the image. Specifically, in the examples of the present application, all the tracklines in the first direction and the second direction may be identified in the image of interest by using image processing techniques such as edge detection, straight line detection (for example, Hough transform), etc. After the tracklines are identified, the crosspoints between the tracklines in the first direction and the tracklines in the second direction are computed. All computed coordinates of crosspoints are checked to see if the coordinates of each crosspoint fall within the boundary of the abnormal region, so as to determine which intersections fall within the marked abnormal region.

In some examples of the present application, for each pair of intersecting tracklines, their coordinates of crosspoints are calculated by a mathematical method (such as solving a linear equation system).

In the examples of the present application, by identifying the position of the crosspoint of the intersecting tracklines, the coordinates of the nucleic acid template in the abnormal region can be accurately determined, thereby facilitating base calling in the image of interest and accurately processing the position of the nucleic acid template in the abnormal region in the image of interest, such that the base calling is more reliable and efficient.

In some examples of the present application, the coordinates of the crosspoints in the abnormal region may be determined by similarity calculation using known coordinates of the crosspoints in a non-abnormal region in the image of interest.

Since the distribution of crosspoints in a field of view (Fov) generally follows a specific principle, the position of the crosspoint in an abnormal region may be more accurately deduced by using the known principle.

On the basis of the structural element arrangement of the image of interest shown in FIG. 5, in the examples of the present application, coordinates of crosspoints (circles in FIG. 5) of all tracklines (black lines in FIG. 5) in the image may be first identified and calculated. By selecting four adjacent crosspoints, a rectangular region, i.e., a block (the rectangular region composed of four circles in FIG. 5), is formed. Using the four adjacent crosspoints, the positions of all the reaction sites (the reaction sites may be, for example, surface-modified nanowells or protrusions for immobilizing the nucleic acid template) in the block can be determined.

It will be appreciated that there are typically only two or one crosspoints for blocks at the edges and corners. In this case, other crosspoints within these blocks are deduced by using adjacent crosspoints at the inner sides of these crosspoints. By knowing two crosspoints in the same line and the number of reaction sites therebetween, the positions of all reaction sites in the line can be located. In addition, coordinates of all reaction sites in the block may be further calculated by using positions of the reaction sites on two parallel tracklines (including longitudinal lines and transverse lines).

In practical applications, the reaction sites are arranged in an array on the surface of the sequencing chip, for example, in a triangular array, a hexagonal array, or an array of other shapes, and the specific calculation method may be different. However, the core idea is to use a known number (e.g., 4, 2, or 1) of crosspoints to calculate the coordinates of the reaction sites within the block.

In the examples of the present application, with the help of the regularity of similarity in the image, the coordinates of the crosspoints in the abnormal region can be accurately calculated by using the known coordinates of the crosspoints of the non-abnormal region in the image of interest, and then the position of the nucleic acid template in the abnormal region in the image of interest can be accurately determined, which is beneficial to reducing the interference with the normal data by the abnormal region in the subsequent base calling process.

In addition, since images of adjacent cycles and adjacent channels are imaged at the same physical location and are corrected to eliminate distortion and chromatic aberration, the images have high similarity. In the examples of the present application, the coordinates of the crosspoints may be extracted from these normal images as a reference to compute crosspoints in the abnormal region.

In some examples of the present application, a set of the coordinates of the crosspoints in the image of interest may be determined; a reference set of the coordinates of the crosspoints in a sequencing image generated by a sequencing cycle previous to the sequencing cycle corresponding to the image of interest is acquired; an offset is calculated by using the set of the coordinates of the crosspoints in the image of interest and the reference set of the coordinates of the crosspoints; and the coordinates of the crosspoints in the abnormal region of the image of interest are determined on the basis of the reference set of the coordinates of the crosspoints and the offset.

Specifically, in the examples of the present application, all coordinates of the crosspoints can be identified and determined in the image of interest. The sequencing image generated by the sequencing cycle previous to the current image of interest is acquired, and the coordinates of the crosspoints therein are extracted and integrated into a reference set of the coordinates of the crosspoints. The sequencing image of the previous cycle is generally considered as normal, or less abnormal. The set of the coordinates of the crosspoints in the image of interest is compared with and aligned to the reference set of the coordinates of the crosspoints, so as to calculate the offset of each crosspoint. This offset reflects the displacement of the crosspoint in the image due to abnormalities or other factors. The coordinates of the crosspointsin the abnormal region are calculated by using the reference set of the coordinates of the crosspoints and the calculated offset. As such, even in the abnormal region, the crosspoints can still be accurately located on the basis of the known reference crosspoints and offset values.

In the examples of the present application, the offset in the abnormal region is calculated by comparing the coordinates of crosspoints in the image of interest and the coordinates of crosspoints in the image of the previous sequencing cycle, so as to accurately locate the coordinates of the crosspoints in abnormal regions and then accurately determine the positions of the nucleic acid template in the abnormal regions of the image of interest, thereby reducing the interference with normal data by abnormal regions during the base calling process.

In some examples of the present application, coordinate differences between reference crosspoints in the reference set of the coordinates of the crosspoints and corresponding crosspoints in the set of the coordinates of the crosspoints in the image of interest may be calculated, and the coordinate difference with the highest frequency of occurrence is taken as the offset.

Since the abnormal region may cause abnormal offsets of some crosspoints, selecting the coordinate difference with the highest frequency may filter these abnormal values, so as to determine an offset that can better represent the overall change of the image and ensure that the most stable and reliable offset value is used for determining the coordinates of the crosspoints in the abnormal region, thereby accurately locating the coordinates of the crosspoints in the abnormal region.

For ease of understanding, an example is used herein for description. For each of different channels (A, C, G, and T channels), a set of the coordinates of the crosspoints is extracted from the image, and the sets are denoted as AcrossPoint, CcrossPoint, GerossPoint, and TcrossPoint. There are 24 crosspoints in each image (pls refer to Fig.5). A set of the coordinates of the crosspoints of a normal sequencing cycle cycN before cycM is selected as a reference set of the coordinates of the crosspoints. The crosspoint in the image generated from cycN is denoted as crossPointN. Similarly, the crosspoint in the image generated from cycM is denoted as crossPointM. For cycN and cycM, the coordinate difference between the corresponding crosspoints in the sets of crossPointN and crossPointM is calculated and denoted as crossPointN-crossPointM. All coordinate differences are summarized, and the difference with the highest frequency of occurrence is selected as the translation between the two images, i.e., the offset. The crosspoint in the image of interest is corrected by using the calculated translation or offset, so as to recalculate the coordinates of crossPointM in the following manner: crossPointM = crossPointN - offset, or crossPointM = crossPointN + offset. It should be noted that CrossPointM may be crosspoints in image acquired from a single channel, or may be a collection of crosspoints in images acquired from multiple channels. Through these steps, even if there are large-area problems such as bubble abnormalities in the cycM image, the accuracy and consistency of subsequent base calling can be ensured through the normal data and offset correction of the previous sequencing cycle.

The invention is further characterized by the following items:
1. A method for detecting an abnormal region in an image, comprising:
   acquiring an image of interest, the image of interest being generated by imaging during the sequencing of a nucleic acid template on a surface of a solid substrate;
   determining a position of the nucleic acid template in the image of interest;
   determining an intensity of a pixel where the nucleic acid template in the image of interest is located on the basis of the position of the nucleic acid template in the image of interest;
   determining whether a signal is present at the position of the nucleic acid template in the image of interest on the basis of the intensity; and
   using a region formed at a position where the nucleic acid template produces no signal in the image of interest as an abnormal region of the image of interest,
   wherein the signal is generated during sequencing of the nucleic acid template.
2. The method according to item 1, wherein
   determining the position of the nucleic acid template in the image of interest comprises:
   acquiring a template image, the template image being an image constructed on the basis of one or more cycles of sequencing of the nucleic acid template and configured for characterizing a position of the nucleic acid template on the surface of the solid substrate; and
   mapping the position of the nucleic acid template to the image of interest to determine the position of the nucleic acid template in the image of interest.
3. The method according to item 1, wherein
   determining the position of the nucleic acid template in the image of interest comprises:
   determining a position of a marking element in the image of interest; and
   determining a position of the nucleic acid template in the image of interest on the basis of the position of the marking element.
4. The method according to item 3, wherein
   the marking element comprises at least one of a trackline and a crosshair.
5. The method according to any one of items 1-4, wherein
   the image of interest comprises one or more sets of corresponding sequencing images generated by performing one or more cycles of sequencing on the nucleic acid template;
   using the region formed at the position where the nucleic acid template produces no signal in the image of interest as the abnormal region of the image of interest comprises:
      using a region formed at a position where the nucleic acid template produces no signal in the one or more sets of sequencing images as the abnormal region.
6. The method according to any one of items 1-5, wherein
   determining whether the signal is present at the position of the nucleic acid template in the image of interest on the basis of the intensity comprises:
   determining a difference between the intensity of the pixel where the nucleic acid template in the image of interest is located and an intensity of a neighborhood pixel thereof;
   comparing the difference with a first threshold; and
   determining whether a signal is present at the position of the nucleic acid template in the image of interest on the basis of the comparison result.
7. The method according to item 6, wherein
   determining whether the signal is present at the position of the nucleic acid template in the image of interest on the basis of the comparison result comprises:
   if the difference is lower than the first threshold, determining that no signal is present at the position of the nucleic acid template in the image of interest.
8. The method according to item 6 or 7, wherein
   the neighborhood comprises an N × M neighborhood,
   both N and M are odd numbers, N ≥ 3, and M ≥ 3.
9. The method according to any one of items 1-8, wherein
   further comprising:
   dividing the image of interest into a number of image blocks;
   determining an evaluation value of each image block; and
   determining whether the image block is an abnormal region of the image of interest on the basis of the evaluation value of the image block,
   wherein the evaluation value is associated with the brightness of the image block.
10. The method according to item 9, wherein
   determining the evaluation value of each image block comprises:
   determining a brightness deviation and a brightness mean of each image block, and determining the evaluation value on the basis of the brightness deviation and the brightness mean.
11. The method according to item 9 or 10, wherein
   determining whether the image block is the abnormal region of the image of interest on the basis of the evaluation value of the image block comprises:
   comparing the evaluation value of the image block with a second threshold; and
   determining whether the image block is an abnormal region of the image of interest on the basis of a comparison result,
   wherein the second threshold is determined on the basis of the global image brightness of the image of interest.
12. The method according to item 11, wherein
   determining whether the image block is the abnormal region of the image of interest on the basis of the comparison result comprises:
   if the evaluation value of the image block is lower than the second threshold, determining that the image block is an abnormal region of the image of interest.
13. The method according to item 12, wherein
   the image of interest comprises one or more sets of corresponding sequencing images generated by performing one or more cycles of sequencing on the nucleic acid template;
   if the evaluation value of the image block is lower than the second threshold, the method further comprises:
      determining whether the evaluation value of the image block in the one or more sets of sequencing images is lower than the second threshold; and
      if yes, determining that the image block is an abnormal region of the image of interest.
14. The method according to any one of items 9-13, further comprising:
   performing an image expansion operation on the abnormal region to expand the boundary of the abnormal region.
15. A method for detecting an abnormal region in an image, comprising:
   acquiring an image of interest, the image of interest being generated by imaging during the sequencing of a nucleic acid template on a surface of a solid substrate, a signal generated during the sequencing of the nucleic acid template being present as a spot in the image of interest, and the brightness of the image of interest being associated with an intensity of the spot;
   dividing the image of interest into a number of image blocks;
   determining an evaluation value of each image block; and
   determining whether the image block is an abnormal region of the image of interest on the basis of the evaluation value of the image block,
   wherein the evaluation value is associated with the brightness of the image block.
16. The method according to item 15, wherein
   determining the evaluation value of each image block comprises:
   determining a brightness deviation and a brightness mean of each image block, and determining the evaluation value on the basis of the brightness deviation and the brightness mean.
17. The method according to item 15 or 16, wherein
   determining whether the image block is the abnormal region of the image of interest on the basis of the evaluation value of the image block comprises:
   comparing the evaluation value of the image block with a second threshold; and
   determining whether the image block is an abnormal region of the image of interest on the basis of a comparison result,
   wherein the second threshold is determined on the basis of the global image brightness of the image of interest.
18. The method according to item 17, wherein
   determining whether the image block is the abnormal region of the image of interest on the basis of the comparison result comprises:
   if the evaluation value of the image block is lower than the second threshold, determining that the image block is an abnormal region of the image of interest.
19. The method according to item 18, wherein
   the image of interest comprises one or more sets of corresponding sequencing images generated by performing one or more cycles of sequencing on the nucleic acid template;
   if the evaluation value of the image block is lower than the second threshold, the method further comprises:
      determining whether the evaluation value of the image block in the one or more sets of sequencing images is lower than the second threshold; and
      if yes, determining that the image block is an abnormal region of the image of interest.
20. The method according to any one of items 15-19, further comprising:
   performing an image expansion operation on the abnormal region to expand the boundary of the abnormal region.
21. The method according to any one of items 15-20, wherein
   further comprising:
   determining a position of the nucleic acid template in the image of interest;
   determining an intensity of a pixel where the nucleic acid template in the image of interest is located on the basis of the position of the nucleic acid template in the image of interest;
   determining whether a signal is present at the position of the nucleic acid template in the image of interest on the basis of the intensity; and
   using a region formed at a position where the nucleic acid template produces no signal in the image of interest as an abnormal region of the image of interest.
22. The method according to item 21, wherein
   determining the position of the nucleic acid template in the image of interest comprises:
   acquiring a template image, the template image being an image constructed on the basis of one or more cycles of sequencing of the nucleic acid template and configured for characterizing a position of the nucleic acid template on the surface of the solid substrate; and
   mapping the position of the nucleic acid template in the template image to the image of interest to determine the position of the nucleic acid template in the image of interest.
23. The method according to item 21, wherein
   determining the position of the nucleic acid template in the image of interest comprises:
   determining a position of a marking element in the image of interest; and
   determining a position of the nucleic acid template in the image of interest on the basis of the position of the marking element.
24. The method according to item 23, wherein
   the marking element comprises at least one of a trackline and a crosshair.
25. The method according to any one of items 21-24, wherein
   the image of interest comprises one or more sets of corresponding sequencing images generated by a process of performing one or more cycles of sequencing on the nucleic acid template;
   using the region formed at the position where the nucleic acid template produces no signal in the image of interest as the abnormal region of the image of interest comprises:
      using a region formed at a position where the nucleic acid template produces no signal in the one or
      more sets of sequencing images as the abnormal region.
26. The method according to any one of items 21-25, wherein
   determining whether the signal is present at the position of the nucleic acid template in the image of interest on the basis of the intensity comprises:
   determining a difference between the intensity of the pixel where the nucleic acid template in the image of interest is located and an intensity of a neighborhood pixel thereof;
   comparing the difference with a first threshold; and
   determining whether a signal is present at the position of the nucleic acid template in the image of interest on the basis of the comparison result.
27. The method according to item 26, wherein
   determining whether the signal is present at the position of the nucleic acid template in the image of interest on the basis of the comparison result comprises:
   if the difference is lower than the first threshold, determining that no signal is present at the position of the nucleic acid template in the image of interest.
28. The method according to item 26 or 27, wherein
   the neighborhood comprises an N × M neighborhood,
   both N and M are odd numbers, N ≥ 3, and M ≥ 3.
29. A method for base calling by processing an abnormal region in an image, comprising:
   determining an abnormal region in the image by detecting with the method according to any one of items 1-28;
   determining a position of the nucleic acid template in the abnormal region in the image of interest; and
   when performing base calling on the basis of the image of interest, zeroing an intensity of a position where the nucleic acid template is located in the abnormal region in the image of interest, and identifying a base type corresponding to the position where the nucleic acid template is located in the abnormal region as an unknown base.
30. The method according to item 29, wherein
   the image of interest comprises tracklines, and the tracklines comprise a number of first tracklines in a first direction and a number of second tracklines in a second direction, wherein the first direction and
   the second direction are not parallel, and intersections of the first tracklines and the second tracklines are defined as crosspoints;
   determining the position of the nucleic acid template in the abnormal region in the image of interest comprises:
      determining coordinates of the crosspoints located in the abnormal region; and
      calculating coordinates of a position of the nucleic acid template in the abnormal region on the basis of the coordinates of the crosspoints in the abnormal region, to determine the position of the nucleic acid template in the abnormal region in the image of interest.
31. The method according to item 30, wherein
   determining the coordinates of the crosspoints located in the abnormal region comprises:
   determining the coordinates of the crosspoints in the abnormal region by similarity calculation using known coordinates of the crosspoints in a non-abnormal region in the image of interest.
32. The method according to item 31, wherein
   determining the coordinates of the crosspoints located in the abnormal region further comprises:
   determining a set of the coordinates of the crosspoints in the image of interest;
   acquiring a reference set of the coordinates of the crosspoints in a sequencing image generated by a sequencing cycle previous to the sequencing cycle corresponding to the image of interest;
   calculating an offset by using the set of the coordinates of crosspoints in the image of interest and the reference set of the coordinates of the crosspoints ; and
   determining the coordinates of the crosspoints in the abnormal region on the basis of the reference set of the coordinates of the crosspoints and the offset.
33. The method according to item 32, wherein
   calculating an offset by using the set of the coordinates of crosspoints in the image of interest and the reference set of the coordinates of the crosspoints comprises:
   calculating coordinate differences between reference crosspoints in the reference set of the coordinates of the crosspoints and corresponding crosspoints in the set of the coordinates of the crosspoints in the image of interest; and
   taking the coordinate difference with the highest frequency of occurrence as the offset.
34. A computer-readable storage medium having a program stored thereon, wherein the program is executable by a processor to implement the method according to any one of items 1-33.
35. A system, comprising:
   the computer-readable storage medium according to item 34; and
   at least one processor configured for executing the program stored in the computer-readable storage medium.
36. An electronic device, comprising at least one processor, at least one memory connected to the processor, and a bus, wherein the processor and the memory communicate with each other through the bus; the processor is configured to invoke program instructions in the memory to execute the method according to any one of items 1-33.
37. A computer program product, comprising: a first instruction for detecting an abnormal region in an image and/or a second instruction for base calling by processing the abnormal region in the image, wherein the first instruction, when executed by a computer, causes the computer to execute the method according to any one of items 1-28, and the second instruction, when executed by a computer, causes the computer to execute the method according to any one of items 29-33.

Although the operations are described in a specific order, it will be appreciated that these operations are not necessarily performed in the shown specific order or in a sequential manner. In certain circumstances, multitasking and parallel processing may be advantageous.

It will be appreciated that the steps described in the method embodiments of the present application may be performed in different orders and/or in parallel. In addition, the method embodiments may include additional steps and/or the listed steps may be omitted, which is not limited in the present application. The examples of the present application provide a computer-readable storage medium having a program stored thereon, where the program is executable by a processor to implement the method for detecting an abnormal region in an image described above and/or the method for base calling by processing an abnormal region in an image described above.

The examples of the present application provide a system, including: the computer-readable storage medium described above, and at least one processor configured for executing the program stored in the computer-readable storage medium.

As shown in FIG. 7, the examples of the present application provide an electronic device 1000. The electronic device 1000 includes at least one processor 1001, at least one memory 1002 connected to the processor 1001, and a bus 1003. The processor 1001 and the memory 1002 communicate with each other through the bus 1003. The processor 1001 is configured to invoke program instructions in the memory 1002 to execute the method for detecting an abnormal region in an image described above and/or the method for base calling by processing an abnormal region in an image described above.

The electronic device herein may be a server, a PC, a PAD, a mobile phone, or the like.

The examples of the present application provide a computer program product, including: a first instruction for detecting an abnormal region in an image and/or a second instruction for base calling by processing the abnormal region in the image. The first instruction, when executed by a computer, causes the computer to execute the method for detecting an abnormal region in an image described above, and the second instruction, when executed by a computer, causes the computer to execute the method for base calling by processing an abnormal region in an image described above.

The processor includes a core, and the core invokes a corresponding program unit from the memory. One or more cores may be provided. By adjusting the core parameters, abnormal regions are identified by analyzing the intensity and the brightness of the image blocks, and the intensity of the abnormal region is zeroed to identify the base as unknown, so as to eliminate the interference by abnormal signals and improve the accuracy of base sequencing.

The present application is illustrated with reference to flowcharts and/or block diagrams of methods, computer-readable storage media, systems, electronic devices, and computer program products according to the examples of the present application. It will be appreciated that each procedure and/or block in the flowcharts and/or block diagrams and a combination of procedures and/or blocks in the flowcharts and/or block diagrams may be implemented by computer program instructions. Such computer program instructions may be provided for a general-purpose computer, a specialized computer, an embedded processor, or a processor of other programmable devices to generate a machine, such that the instructions executed by the computer or the processor of the another programmable device generate an apparatus for implementing a specific function in one or more procedures in the flowcharts and/or in one or more blocks in the block diagrams.

In a typical configuration, the electronic device includes one or more processors (CPUs), a memory, and a bus. The electronic device may further include an input/output interface, a network interface, and the like.

The memory may include a non-persistent memory, a random access memory (RAM), a non-volatile memory, and/or the like, in a computer-readable medium, for example, a read-only memory (ROM) or a flash memory (flash RAM). The memory includes at least one storage chip. The memory is an example of the computer-readable medium.

The computer-readable medium includes persistent, non-persistent, removable, and non-removable media that can store information by using any method or technology. The information may be a computer-readable instruction, a data structure, a program module, or other data. Examples of the computer storage medium include, but are not limited to, a phase change random access memory (PRAM), a static random access memory (SRAM), a dynamic random access memory (DRAM), other types of random access memories (RAMs), a read-only memory (ROM), an electrically erasable programmable read-only memory (EEPROM), a flash memory or other memory technologies, a compact disc read-only memory (CD-ROM), a digital versatile disc (DVD) or other optical storages, a cassette magnetic tape, a tape disk storage or other magnetic storage devices, or any other non-transmission media configured to store information that can be accessed by a computing device. On the basis of the definition herein, the computer-readable medium does not include transitory computer-readable media (transitory media), for example, a modulated data signal and carrier.

In the description of the present application, it should be noted that the orientational or positional relationship indicated by the terms "upper", "lower", "front", "rear", "left", and "right" is based on the orientational or positional relationship illustrated in the drawings, and is merely for the convenience and ease of illustrating the present application, rather than indicating or implying that the noted position or element must have a specific orientation or be configured or operated in the specific orientation. Such relationships should not be construed as limiting the present application.

It should be noted that relational terms such as first and second herein are only used to distinguish one entity or procedure from another entity or procedure, and do not necessarily require or imply any such actual relationship or order between these entities or procedures. It should further noted that the terms "include", "comprise", or their any other variants are intended to encompass a non-exclusive inclusion, such that a process, a method, a product, or a device that includes a list of elements not only includes those elements but also includes other elements which are not expressly listed, or further includes elements inherent to such a process, method, product, or device. An element defined by "comprising a ..." does not, without further constraints, exclude the presence of additional identical elements in the process, method, product, or device that includes the element.

Those skilled in the art will appreciate that the examples of the present application may be provided as a method, a system, or a computer program product. Therefore, the present application may be in the form of a hardware-only example, a software-only example, or an example with a combination of software and hardware. In addition, the present application may be in the form of a computer program product that is implemented on one or more computer-usable storage media (including but not limited to a magnetic disk memory, a CD-ROM, an optical memory, and the like) that include a computer-usable program code.

The above descriptions are merely examples of the present application and are not intended to limit the present application. For those skilled in the art, the present application may have various modifications and variations. Any modification, equivalent substitution, or improvement made without departing from the spirit and principle of the present application shall fall within the scope of the present application.

## Claims

1. A method for detecting an abnormal region in an image, comprising:
acquiring an image of interest, the image of interest being generated by imaging during the sequencing of a nucleic acid template on a surface of a solid substrate;
determining a position of the nucleic acid template in the image of interest;
determining an intensity of a pixel where the nucleic acid template in the image of interest is located on the basis of the position of the nucleic acid template in the image of interest;
determining whether a signal is present at the position of the nucleic acid template in the image of interest on the basis of the intensity; and
using a region formed at a position where the nucleic acid template produces no signal in the image of interest as an abnormal region of the image of interest,
wherein the signal is generated during sequencing of the nucleic acid template.

2. The method according to claim 1, wherein
determining the position of the nucleic acid template in the image of interest comprises:
acquiring a template image, the template image being an image constructed on the basis of one or more cycles of sequencing of the nucleic acid template and configured for characterizing a position of the nucleic acid template on the surface of the solid substrate; and
mapping the position of the nucleic acid template to the image of interest to determine the position of the nucleic acid template in the image of interest.

3. The method according to claim 1, wherein
determining the position of the nucleic acid template in the image of interest comprises:
determining a position of a marking element in the image of interest; and
determining a position of the nucleic acid template in the image of interest on the basis of the position of the marking element;
optionally, the marking element comprises at least one of a trackline and a crosshair.

4. The method according to any one of claims 1-3, wherein
the image of interest comprises one or more sets of corresponding sequencing images generated by performing one or more cycles of sequencing on the nucleic acid template;
using the region formed at the position where the nucleic acid template produces no signal in the image of interest as the abnormal region of the image of interest comprises:
using a region formed at a position where the nucleic acid template produces no signal in the one or more sets of sequencing images as the abnormal region.

5. The method according to any one of claims 1-4, wherein
determining whether the signal is present at the position of the nucleic acid template in the image of interest on the basis of the intensity comprises:
determining a difference between the intensity of the pixel where the nucleic acid template in the image of interest is located and an intensity of a neighborhood pixel thereof;
comparing the difference with a first threshold; and
determining whether a signal is present at the position of the nucleic acid template in the image of interest on the basis of the comparison result;
if the difference is lower than the first threshold, determining that no signal is present at the position of the nucleic acid template in the image of interest;
optionally, the neighborhood comprises an N × M neighborhood,
both N and M are odd numbers, N ≥ 3, and M ≥ 3.

6. The method according to any one of claims 1-5, wherein
further comprising:
dividing the image of interest into a number of image blocks;
determining an evaluation value of each image block; and
determining whether the image block is an abnormal region of the image of interest on the basis of the evaluation value of the image block,
wherein the evaluation value is associated with the brightness of the image block.

7. The method according to claim 6, wherein
determining the evaluation value of each image block comprises:
determining a brightness deviation and a brightness mean of each image block, and determining the evaluation value on the basis of the brightness deviation and the brightness mean.

8. The method according to claim 6 or 7, wherein
determining whether the image block is the abnormal region of the image of interest on the basis of the evaluation value of the image block comprises:
comparing the evaluation value of the image block with a second threshold; and
determining whether the image block is an abnormal region of the image of interest on the basis of a comparison result,
wherein the second threshold is determined on the basis of the global image brightness of the image of interest.

9. The method according to claim 8, wherein
determining whether the image block is the abnormal region of the image of interest on the basis of the comparison result comprises:
if the evaluation value of the image block is lower than the second threshold, determining that the image block is an abnormal region of the image of interest.

10. The method according to claim 9, wherein
the image of interest comprises one or more sets of corresponding sequencing images generated by performing one or more cycles of sequencing on the nucleic acid template;
if the evaluation value of the image block is lower than the second threshold, the method further comprises:
determining whether the evaluation value of the image block in the one or more sets of sequencing images is lower than the second threshold; and
if yes, determining that the image block is an abnormal region of the image of interest;
optionally, further comprising:
performing an image expansion operation on the abnormal region to expand the boundary of the abnormal region.

11. A method for detecting an abnormal region in an image, comprising:
acquiring an image of interest, the image of interest being generated by imaging during the sequencing of a nucleic acid template on a surface of a solid substrate, a signal generated during the sequencing of the nucleic acid template being present as a spot in the image of interest, and the brightness of the image of interest being associated with an intensity of the spot;
dividing the image of interest into a number of image blocks;
determining an evaluation value of each image block; and
determining whether the image block is an abnormal region of the image of interest on the basis of the evaluation value of the image block,
wherein the evaluation value is associated with the brightness of the image block.

12. The method according to claim 11, wherein
determining the evaluation value of each image block comprises:
determining a brightness deviation and a brightness mean of each image block, and determining the evaluation value on the basis of the brightness deviation and the brightness mean.

13. The method according to claim 11 or 12, wherein
determining whether the image block is the abnormal region of the image of interest on the basis of the evaluation value of the image block comprises:
comparing the evaluation value of the image block with a second threshold; and
determining whether the image block is an abnormal region of the image of interest on the basis of a comparison result,
wherein the second threshold is determined on the basis of the global image brightness of the image of interest;
if the evaluation value of the image block is lower than the second threshold, determining that the image block is an abnormal region of the image of interest;
optionally, the image of interest comprises one or more sets of corresponding sequencing images generated by performing one or more cycles of sequencing on the nucleic acid template;
if the evaluation value of the image block is lower than the second threshold, the method further comprises:
determining whether the evaluation value of the image block in the one or more sets of sequencing images is lower than the second threshold; and
if yes, determining that the image block is an abnormal region of the image of interest;
optionally, further comprising:
performing an image expansion operation on the abnormal region to expand the boundary of the abnormal region.
optionally, further comprising:
determining a position of the nucleic acid template in the image of interest;
determining an intensity of a pixel where the nucleic acid template in the image of interest is located on the basis of the position of the nucleic acid template in the image of interest;
determining whether a signal is present at the position of the nucleic acid template in the image of interest on the basis of the intensity; and
using a region formed at a position where the nucleic acid template produces no signal in the image of interest as an abnormal region of the image of interest;
optionally, determining the position of the nucleic acid template in the image of interest comprises:
acquiring a template image, the template image being an image constructed on the basis of one or more cycles of sequencing of the nucleic acid template and configured for characterizing a position of the nucleic acid template on the surface of the solid substrate; and
mapping the position of the nucleic acid template in the template image to the image of interest to determine the position of the nucleic acid template in the image of interest;
optionally, determining the position of the nucleic acid template in the image of interest comprises:
determining a position of a marking element in the image of interest; and
determining a position of the nucleic acid template in the image of interest on the basis of the position of the marking element;
optionally, the marking element comprises at least one of a trackline and a crosshair.
optionally, the image of interest comprises one or more sets of corresponding sequencing images generated by a process of performing one or more cycles of sequencing on the nucleic acid template; using the region formed at the position where the nucleic acid template produces no signal in the image of interest as the abnormal region of the image of interest comprises:
using a region formed at a position where the nucleic acid template produces no signal in the one or
more sets of sequencing images as the abnormal region;
optionally, determining whether the signal is present at the position of the nucleic acid template in the image of interest on the basis of the intensity comprises:
determining a difference between the intensity of the pixel where the nucleic acid template in the image of interest is located and an intensity of a neighborhood pixel thereof;
comparing the difference with a first threshold; and
determining whether a signal is present at the position of the nucleic acid template in the image of interest on the basis of the comparison result;
optionally, determining whether the signal is present at the position of the nucleic acid template in the image of interest on the basis of the comparison result comprises:
if the difference is lower than the first threshold, determining that no signal is present at the position of the nucleic acid template in the image of interest;
optionally, the neighborhood comprises an N × M neighborhood,
both N and M are odd numbers, N ≥ 3, and M ≥ 3.

14. A method for base calling by processing an abnormal region in an image, comprising:
determining an abnormal region in the image by detecting with the method according to any one of claims 1-13;
determining a position of the nucleic acid template in the abnormal region in the image of interest; and
when performing base calling on the basis of the image of interest, zeroing an intensity of a position where the nucleic acid template is located in the abnormal region in the image of interest, and identifying a base type corresponding to the position where the nucleic acid template is located in the abnormal region as an unknown base.

15. The method according to claim 14, wherein
the image of interest comprises tracklines, and the tracklines comprise a number of first tracklines in a first direction and a number of second tracklines in a second direction, wherein the first direction and the second direction are not parallel, and intersections of the first tracklines and the second tracklines are defined as crosspoints;
determining the position of the nucleic acid template in the abnormal region in the image of interest comprises:
determining coordinates of the crosspoints located in the abnormal region; and
calculating coordinates of a position of the nucleic acid template in the abnormal region on the basis of the coordinates of the crosspoints in the abnormal region, to determine the position of the nucleic acid template in the abnormal region in the image of interest;
optionally, determining the coordinates of the crosspoints located in the abnormal region comprises:
determining the coordinates of the crosspoints in the abnormal region by similarity calculation using known coordinates of the crosspoints in a non-abnormal region in the image of interest;
optionally, determining the coordinates of the crosspoints located in the abnormal region further comprises:
determining a set of the coordinates of the crosspoints in the image of interest;
acquiring a reference set of the coordinates of the crosspoints in a sequencing image generated by a sequencing cycle previous to the sequencing cycle corresponding to the image of interest;
calculating an offset by using the set of the coordinates of crosspoints in the image of interest and the reference set of the coordinates of the crosspoints ; and
determining the coordinates of the crosspoints in the abnormal region on the basis of the reference set of the coordinates of the crosspoints and the offset;
optionally, calculating an offset by using the set of the coordinates of crosspoints in the image of interest and the reference set of the coordinates of the crosspoints comprises:
calculating coordinate differences between reference crosspoints in the reference set of the coordinates of the crosspoints and corresponding crosspoints in the set of the coordinates of the crosspoints in the image of interest; and
taking the coordinate difference with the highest frequency of occurrence as the offset.
